# EUROPÄISCHE PATENTSCHRIFT

(11) **EP 0 360 180 B1**
(45) Veröffentlichungstag und Bekanntmachung des Hinweises auf die Patenterteilung: **14.06.1995**
(21) Anmeldenummer: 89117187.8
(22) Anmeldetag: 16.09.1989
(51) Int. Cl.: C08J 9/00, A61L 15/32, A61K 47/42, C08L 89/06

(54) **Verfahren zur Herstellung von Kollagenschäumen in Form endloser Bänder und Verwendung in Medizin, Kosmetik und Hygiene**
Process for making collagenous foams in the shape of continuous tapes, and their use in medicine, cosmetics and hygiene
Procédé de fabrication de mousses de collagène sous forme de bandes sans fin et leur application en médecine, cosmétiques et hygiène

(30) Priorität: 22.09.1988 DE 3832162
(43) Veröffentlichungstag der Anmeldung: 28.03.1990
(73) Patentinhaber: LTS Lohmann Therapie-Systeme GmbH & Co. KG, 56513 Neuwied (DE)
(72) Erfinder: Anhäuser, Dieter, D-5451 Melsbach (DE); Roreger, Michael, D-5450 Neuwied 22 (DE)
(74) Vertreter: Flaccus, Rolf-Dieter, Dr.

(56) Entgegenhaltungen:
- EP-A- 0 209 726
- EP-A- 0 314 109
- GB-A- 1 019 711
- US-A- 3 939 831
- US-A- 4 066 083

## Beschreibung

Die Erfindung betrifft ein Verfahren zur Herstellung von Kollagenschäumen in Form endloser Bänder und ihre Verwendung in Medizin, Kosmetik und Hygiene.

Es ist bekannt, daß in Medizin, Kosmetik und Hygiene Kollagenschäume oder -fasergeflechte mit hervorragender Haut- und Schleimhautverträglichkeit Verwendung finden zum einen wegen der Eigenwirkung des Kollagens, zum anderen wegen der guten Saug- und Polstereigenschaften von Kollagenschäumen, so z.B. in der Medizin zur lokalen Blutstillung, als Wundabdeckung mit wundheilungsfördernden Eigenschaften, als Füllmaterial für Knochenhohlräume oder als resorbierbarer Wirkstoffträger für Implantate.

Verfahren zur Herstellung solcher Kollagenschäume mit Schwamm- oder Vliesstruktur für die genannten Zwecke durch Gefriertrocknung von Kollagenlösungen oder -dispersionen sind seit langem bekannt (z.B. EP 209 726 Beutler/Ebinger/Lindner, DE-OS 32 03 957 Eckmayer, US-PS 3,157,524 Artandi, DE 29 43 520 Berg/Eckmayer, DE-OS 33 15 678 Cioca, DE 18 11 290 Chvapil). Bei diesen Verfahren werden Kollagenlösungen oder -dispersionen in Behältnisse gegossen und in diesen Behältnissen gefriergetrocknet. Durch Form und Abmessungen der Behältnisse sind dabei Form und Abmessungen der gefriergetrockneten Kollagenschäume vorgegeben. Diese Schäume werden entweder direkt in den Behältnissen oder nach Herausnahme aus den Behältnissen weiter konfektioniert. Nachteil dieser Verfahren ist, daß durch die Einzelherstellung jedes Schaums alle weiteren Verarbeitungsschritte nach der Gefriertrocknung sehr zeitaufwendig und damit kostenaufwendig sind. Die gefertigten Stückzahlen des Endprodukts pro Zeiteinheit sind nur gering. Eine andere Möglichkeit besteht darin Kollagenlösungen oder -dispersionen unter Zusatz von Gerb- oder Vernetzungsmitteln in entsprechenden Behältnissen großflächig zu lyophilisieren und anschließend aus den gefriergetrockneten Platten durch Spalten, Schneiden und/oder Stanzen Kollagenschäume eines bestimmten Formats herzustellen. Nachteilig ist bei diesen Verfahren zum einen, daß die großflächigen Schäume zur Erzielung einer für die weitere Verarbeitung notwendigen Festigkeit durch Gerbung oder Vernetzung des Kollagens stabilisiert werden müssen, zum anderen, daß die Weiterverarbeitung der großflächigen Schäume diskontinuierlich erfolgt und ebenfalls einen hohen Personal- und Zeitaufwand erfordert.

Aus der US-A-4,066,083 ist ein Verfahren zur Herstellung eines stabilen, chirurgischen Kollagenproduktes mit filzartiger oder vliesstoffartiger Struktur bekannt, mit hämostatischer Wirkung, einer hohen Absorptionskapazität für Körperflüssigkeiten, welches die Geweberegeneration begünstigt, hochresorptiv ist, eine nicht-antigene Aktivität besitzt und optimale mechanische Eigenschaften bzw. Eignung zur Applikation auf Wundstellen oder Einbringung in weunden oder in Knochenhöhlen aufweist. Zur Herstellung wird eine Kollagenlösung in runden Schalen gefriergetrocknet, wobei 0,5 cm dicke Kollagenscheiben erhalten und diese einzeln in Polyethylenbeuteln verpackt, versiegelt und anschließend mit Gamma-Strahlung sterilisiert werden.

Aufgabe der Erfindung ist es, ein Verfahren zu entwickeln, das die Verarbeitung von hochreinem, unvernetztem Kollagen in gefriergetrockneter Form zu bestimmten medizinischen Produkten (Wundschnellverband mit durchlaufendem Wundkissen aus Kollagen, Wundschnellverband mit zentralem Wundkissen aus Kollagen, hämostypische Pflaster, Tupfer zur Blutstillung, Wundauflagen) in einem wirtschaftlichen, möglichst kontinuierlichen Verfahren mit hohem Stückumsatz pro Zeiteinheit ermöglicht.

Zur Lösung der Aufgabe entsprechend den Merkmalen des Hauptanspruchs wird mit der Erfindung vorgeschlagen, daß
a) gelöstes oder dispergiertes Kollagen aufbereitet und in Scheibenform gegossen wird, wobei zur Stabilisierung des Schaumes bei der Aufbereitung des Kollagens eine Spaltung der intermolekularen Bindungen des Kollagens so gesteuert wird, daß nur eine geringe Anzahl intermolekularer Bindungen gespalten und überwiegend höhermolekulare, wasserunlösliche Kollagenaggregate mit helikaler Struktur der Moleküle erhalten bleiben, die einen hohen natürlichen Vernetzungsgrad des Kollagens und damit Kollagenschäume mit hoher mechanischer Festigkeit ergeben, und daß die Kollagenkonzentration in der Lösung oder Dispersion 0,1 bis 5 Gew.-%, vorzugsweise 0,5 bis 2 Gew.-% beträgt,
b) die in die Scheibenform gefüllte Kollagenlösung oder -dispersion zur Erzielung eines homogenen Schwamms mit sehr feinen Poren auf Temperaturen von -50°C oder niedriger schockgefroren und anschließend unter Vakuum gefriergetrocknet wird, wobei die Trocknungsdauer bis zur Erreichung einer Produkttemperatur von ca. 30°C je nach Schichtdicke des Lyophilisats zwischen 12 h und 60 h liegt
c) der gefriergetrocknete Kollagenschaum vor der weiteren Verarbeitung auf eine genau definierte Dicke gebracht wird und
d) maschinell aus den gefriergetrockneten Scheiben des Kollagens durch einen Schälvorgang Endlosbänder hergestellt werden
Auf diese Weise ist mit großem Vorteil eine wirtschaftliche und kontinuierliche Herstellung von Kollagenschäumen in Form endloser Bänder möglich, wodurch die Herstellung von Endprodukten wie Wundschnellverbände, Wundkissen, hämostypische Pflaster, Tupfer zur Blutstillung, Wundauflagen etc. außerordentlich rationalisiert wird.
Eine Ausgestaltung des Verfahrens ist entsprechend der Lehre von Anspruch 2 vorgesehen. Dadurch gelingt es, durch den hohen natürlichen Vernetzungsgrad des Kollagens den Schaum mit Vorteil zu stabilisieren, ohne daß zusätzliche Manipulationen zur Verfestigung des Schaums, wie z.B. Gerbung oder Vernetzung überflüssig werden und die ursprüngliche Quartärstruktur des Kollagens, wie sie in der Haut vorliegt, weitgehend erhalten bleibt.

Eine Ausgestaltung sieht vor, daß die Kollagenkonzentration in der Lösung oder Dispersion 0,1 bis 5 Gew.-%, vorzugsweise 0,5 bis 2 Gew.-% beträgt.

Mit weiteren Ausgestaltungen ist vorgesehen, daß die Kollagenlösung oder -dispersion Wirk- und/oder Hilfsstoffe enthält, sowie, daß die Kollagenlösung oder -dispersion steril ist.
Eine Ausgestaltung sieht vor, daß die Scheibenform, in die die Kollagenlösung oder -dispersion gefüllt wird, von beliebiger, vorzugsweise runder Gestalt ist. Die Scheibenform kann eine beliebige Höhe zwischen 0,5 und 10 cm, vorzugsweise zwischen 1 und 4 cm und einem beliebigen Durchmesser, vorzugsweise zwischen 50 und 70 cm, haben.
Sehr vorteilhaft läßt sich der Schälvorgang der Scheibe durchführen, wenn diese eine zentrale Aussparung, vorzugsweise von runder Gestalt, mit eine beliebigen Fläche, vorzugsweise zwischen 1 cm² und 80 cm², enthält, so daß der Kollagenschaum nach der Gefriertrocknung ein mittiges Loch aufweist, dessen Durchmesser vorzugsweise etwas kleiner ist als der Durchmesser des Wickelkerns der Schälmaschine ist, damit die Kollagenschäume während des Schälvorgangs nicht verrutschen. Die Kollagenzubereitung in der Scheibenform wird in einem Gefrierschrank oder im Lyophilisator selbst mit hoher Temperaturerniedrigungsrate auf Temperaturen von kleiner gleich -50°C schockgefroren zur Bildung möglichst kleiner Eiskristalle, so daß die Porenstruktur in dem fertigen Schaum fein und gleichmäßig ist. Die tiefgefrorene Kollagenzubereitung wird dann unter Vakuum gefriergetrocknet, wobei die Trocknungsdauer bis zur Erreichen einer Produkttemperatur von ca. 30°C je nach Schichtdicke des Lyophilisats zwischen 12 h und 60 h liegt.

Da die Kollagenschäume nach der Gefriertrocknung häufig eine unregelmäßige Oberflächenstruktur aufweisen, werden diese Schäume vor der weiteren Verarbeitung auf eine definierte Dicke abgerichtet.

Falls bei der Abfüllung eine Scheibenform ohne zentrale Aussparung verwendet wird, kann nach der Gefriertrocknung aus dem Kollagenschaum ein mittiges Loch herausgestanzt oder geschnitten werden. Auch kann ein scheibenförmiger Kollagenschaum oder mehrere scheibenförmige Kollagenschäume nebeneinander vertikal auf einen Wickelkern geschoben werden. Auf diese Weise können auf einer Bandschälmaschine von der oder den Scheiben fortlaufende Bänder beliebiger Dicke, vorzugsweise von 1 bis 5 mm Dicke, abgeschält werden. Außer mit einem bekannten Bandschälverfahren können aus den Kollagenscheiben auch mittels anderer bekannter Techniken, vorzugsweise durch Laserstrahl-Schnitt fortlaufende Bänder hergestellt werden.

Hierbei ist weiter vorgesehen, daß die so hergestellten Kollagenbänder zu Rollen aufgewickelt oder gefaltet und in Behältnisse abgestapelt werden.
Mit Vorteil können aber auch die so hergestellten Kollagenbänder in einem der Bandherstellung direkt nachgeschalteten Arbeitsschritt weiterverarbeitet werden.
Beispielsweise können von dem erfindungsgemäß hergestellten Kollagenband mittels Querschneider einzelne Abschnitte jeweils definierter Größe abgeteilt werden, die einzeln oder zu mehreren konfektioniert oder aufgerollt in einem Spender, der vorzugsweise eine Abreißkante aufweist, untergebracht werden.

### Kollagen, Nativkollagen, Kollagenlösung, -dispersion Zugabe von Wirkststoffen

Das erfindungsgemäß verwendete Kollagen stammt vorzugsweise aus junger Kalbshaut, die zunächst enthaart und entfettet wird. Danach werden lösliche Kollagene und die nichtkollagenen Bestandteile des Bindegewebes extrahiert. Bei dem zurückbleibenden, in Wasser unlöslichen Kollagen werden die Telopeptide, die für die antigenen Eigenschaften des Kollagens verantwortlich sind, abgespalten, und durch ein entsprechend gesteuertes Verfahren ein definierter Teil der intermolekularen Kollagenbindungen gespalten. Die intramolekularen Bindungen des Kollagens werden nicht angegriffen, so daß die helikale Struktur des Moleküls intakt bleibt.

Diese Spaltung kann so geführt werden, daß ein Großteil des Kollagens wasserlöslich gemacht wird, so daß nach Filtration oder Zentrifugation eine von unlöslichem Kollagen befreite Lösung erhalten wird. Wird die Spaltung so gesteuert, daß nur eine geringe Anzahl intermolekularer Bindungen gespalten wird, werden überwiegend höhermolekulare, wasserunlösliche Kollagenaggregate erhalten, die in Wasser dispergiert werden. Eine stabile Dispersion enthält bis zu etwa 5 Gew.-%, vorzugsweise bis zu 2 Gew.-%, Kollagen in gleichmäßiger Verteilung. Zur Herstellung von gefriergetrockneten Kollagenschäumen mit hoher mechanischer Festigkeit werden vorzugsweise Dispersionen mit sehr hohem Anteil mit hochmolekularen Aggregaten verwendet, da durch den hohen natürlichen Vernetzungsgrad des Kollagens der Schaum stabilisiert wird. Das hat den Vorteil, daß zusätzliche Manipulationen zur Verfestigung des Schaums, wie z.B. Gerbung oder Vernetzung, überflüssig werden und die ursprüngliche Quartärstruktur des Kollagens, wie sie in der Haut vorliegt, weitgehend erhalten bleibt.
In die Kollagenzubereitung (Lösung oder Dispersion) können weitere, vorzugsweise medizinische Wirkstoffe, z.B. Antiseptika oder Antibiotika, sowie weitere Hilfsstoffe eingearbeitet werden. Soll die Kollagenzubereitung zur Herstellung von sterilen Produkten für medizinische Zwecke verwendet werden, so wird das Kollagenisolierungsverfahren so geführt, daß die erhaltene Kollagenzubereitung steril ist.

### Abfüllen, Gefriertrocknen, Scheibenformen Vorbehandlung der Scheiben

Die Kollagenzubereitung wird in Scheibenformen von beliebiger, vorzugsweise von runder Gestalt mit einer beliebigen Höhe zwischen 0,5 und 10 cm, vorzugsweise zwischen 1 und 4 cm, und einem beliebigen Durchmesser, vorzugsweise zwischen 50 und 70 cm, eingefüllt, wobei die Füllhöhe der Kollagenzubereitung in der Form so gewählt wird, daß die Zubereitung beim Transport der Form und beim Einbringen in den Lyophilisator nicht über den Rand der Form schwappt. Als Scheibe wird im Sinne der Erfindung eine Form verstanden, deren Dicke klein im Verhältnis zu ihrem Durchmesser ist. Soll der nach der Gefriertrocknung erhaltene Kollagenschwamm in einem späteren Verfahrensschritt auf einer Schälmaschine weiterverarbeitet werden, so wird die Kollagenzubereitung vorzugsweise in eine Scheibenform gefüllt, die eine zentrale Aussparung von beliebiger, vorzugsweise runder Gestalt mit einem beliebigen Durchmesser, vorzugsweise zwischen 1 und 10 cm, enthält. Der Kollagenschaum weist in diesem Fall nach der Gefriertrocknung ein mittiges Loch auf, dessen Durchmesser vorzugsweise etwas kleiner als der Durchmesser des Wickelkerns der Schälmaschine ist, damit die Kollagenschäume während des Schälvorgangs nicht verrutschen. Die Kollagenzubereitung in der Scheibenform wird in einem Gefrierschrank oder im Lyophilisator selbst mit hoher Temperaturerniedrigungsrate auf Temperaturen von kleiner gleich -50°C schockgefroren zur Bildung möglichst kleiner Eiskristalle, so daß die Porenstruktur in dem fertigen Schaum fein und gleichmäßig ist. Die tiefgefrorene Kollagenzubereitung wird dann unter Vakuum gefriergetrocknet, wobei die Trocknungsdauer bis zur Erreichung einer Produkttemperatur von ca. 30°C je nach Schichtdicke des Lyophilisats zwischen 12 h und 60 h liegt.

Da die Kollagenschäume nach der Gefriertrocknung häufig eine unregelmäßige Oberflächenstruktur aufweisen, werden diese Schäume vor der weiteren Verarbeitung auf eine definierte Dicke abgerichtet. Ferner wird, falls zur Gefriertrocknung keine Form mit zentraler Aussparung verwendet wurde und die weitere Verarbeitung mittels Schälverfahren erfolgen soll, aus dem Kollagenschaum ein mittiges Loch geschnitten oder gestanzt.

### Beispiel:

In Scheibenformen (kreisrunde Bodenplatte mit 580 mm Durchmesser, außen umlaufender Steg mit 45 mm Höhe, innen kreisrunde Aussparung von 83 mm Durchmesser und Steghöhe von 45 mm) werden jeweils 9,0 kg Nativkollagendispersion 0,75 % gegossen (Füllhöhe in der Form ca. 35 mm). Die Dispersion in der Form wurde im Lyophilisator schockgefroren und anschließend ca. 48 h vakuumgefriergetrocknet. Nach Beendigung der Trocknung wurden radförmige Kollagenscheiben von 25 mm Dicke erhalten. Mehrere dieser Kollagenscheiben wurden nebeneinander horizontal mit der zentralen Bohrung auf die gerippte Schälachse einer Bandmesser-Schälmaschine geschoben. Von den Kollagenscheiben wurden dann Bänder von ca. 3mm Dicke auf ca. 70 m Länge abgeschält und automatisch auf einem Wickelkern aufgewickelt.
Zur Herstellung eines Wundschnellverbands mit durchgehendem Wundkissen wird das Kollagenband mit 25 mm Breite auf einem Konfektionierungsautomaten auf einen selbstklebend ausgerüsteten textilen Träger mittig aufkaschiert, anschließend abgedeckt, auf das Format 60 X 20 mm gestanzt und konfektioniert.

## Patentansprüche

1. Verfahren zur Herstellung von Kollagenschäumen in Form endloser Bänder, dadurch gekennzeichnet, daß
a) gelöstes oder dispergiertes Kollagen aufbereitet und in Scheibenform gegossen wird, wobei zur Stabilisierung des Schaumes bei der Aufbereitung des Kollagens eine Spaltung der intermolekularen Bindungen des Kollagens so gesteuert wird, daß nur eine geringe Anzahl intermolekularer Bindungen gespalten und überwiegend höhermolekulare, wasserunlösliche Kollagenaggregate mit helikaler Struktur der Moleküle erhalten bleiben, die einen hohen natürlichen Vernetzungsgrad des Kollagens und damit Kollagenschäume mit hoher mechanischer Festigkeit ergeben, und daß die Kollagenkonzentration in der Lösung oder Dispersion 0,1 bis 5 Gew.-%, vorzugsweise 0,5 bis 2 Gew.-% beträgt,
b) die in die Scheibenform gefüllte Kollagenlösung oder -dispersion zur Erzielung eines homogenen Schwamms mit sehr feinen Poren auf Temperaturen von -50°C oder niedriger schockgefroren und anschließend unter Vakuum gefriergetrocknet wird, wobei die Trocknungsdauer bis zur Erreichung einer Produkttemperatur von ca. 30 °C je nach Schichtdicke des Lyophilisats zwischen 12 h und 60 h liegt;
c) der gefriergetrocknete Kollagenschaum vor der weiteren Verarbeitung auf eine genau definierte Dicke gebracht wird und
d) maschinell aus den gefriergetrockneten Scheiben des Kollagens durch eine Schälvorgang Endlosbänder hergestellt werden.

2. Verfahren gemäß Anspruch 1 , dadurch gekennzeichnet, daß die Kollagenlösung oder -dispersion Wirk- und/oder Hilfsstoffe enthält.

3. Verfahren gemäß Anspruch 1 bis 2, dadurch gekennzeichnet, daß die Kollagenlösung oder -dispersion steril ist.

4. Verfahren gemäß Anspruch 1 , dadurch gekennzeichnet, daß die Scheibenform, in die die Kollagenlösung oder -dispersion gefüllt wird, von beliebiger, vorzugsweise runder Gestalt ist.

5. Verfahren gemäß Anspruch 1 , dadurch gekennzeichnet, daß die Scheibenform eine beliebige Höhe zwischen 0,5 und 10 cm, vorzugsweise zwischen 1 und 4 cm und einen beliebigen Durchmesser, vorzugsweise zwischen 50 und 70 cm, hat.

6. Verfahren gemäß Anspruch 1 bis 5, dadurch gekennzeichnet, daß die Scheibenform eine zentrale Aussparung, vorzugsweise von runder Gestalt, mit einer beliebigen Fläche, vorzugsweise zwischen 1 cm² und 80 cm², enthält, so daß der Kollagenschaum nach der Gefriertrocknung ein mittiges Loch aufweist.

7. Verfahren gemäß Anspruch 1 bis 6, dadurch gekennzeichnet, daß falls bei der Abfüllung eine Scheibenform ohne zentrale Ausparung verwendet wird, nach der Gefriertrocknung aus dem Kollagenschaum ein mittiges Loch herausgestanzt oder geschnitten wird.

8. Verfahren gemäß Anspruch 1 bis 7, dadurch gekennzeichnet, daß ein scheibenförmiger Kollagenschaum oder mehrere scheibenförmige Kollagenschäume nebeneinander vertikal auf einen Wickelkern geschoben wird bzw. werden.

9. Verfahren gemäß Anspruch 1 bis 8, dadurch gekennzeichnet, daß auf einer Bandschälmaschine von der oder den Scheiben fortlaufende Bänder beliebiger Dicke, vorzugsweise von 1 bis 5 mm Dicke, abgeschält werden.

10. Verfahren gemäß Anspruch 1 bis 9, dadurch gekennzeichnet, daß aus den Kollagenscheiben mittels anderer bekannter Techniken, vorzugsweise Laserstrahlschnitt, fortlaufende Bänder hergestellt werden.

11. Verfahren gemäß Anspruch 1 bis 10, dadurch gekennzeichnet, daß die so hergestellten Kollagenbänder zu Rollen aufgewickelt werden.

12. Verfahren gemäß Anspruch 1 bis 10, dadurch gekennzeichnet, daß die so hergestellten Kollagenbänder gefaltet und in Behältnisse abgestapelt werden.

13. Verfahren gemäß Anspruch 1 bis 10, dadurch gekennzeichnet, daß die so hergestellten Kollagenbänder in einem der Bandherstellung direkt nachgeschalteten Arbeitsschritt weiterverarbeitet werden.

14. Verfahren gemäß Anspruch 1 bis 13, dadurch gekennzeichnet, daß von dem erfindungsgemäß hergestellten Kollagenband mittels Querschneider einzelne Abschnitte jeweils definierter Größe abgeteilt werden, die einzeln oder zu mehreren konfektioniert oder aufgerollt in einem Spender, der vorzugsweise eine Abreißkante aufweist, untergebracht werden.

15. Verfahren gemäß Anspruch 1 bis 13, dadurch gekennzeichnet, daß aus dem erfindungsgemäß hergestellten Kollagenband und anderen bahnförmigen Materialien durch bekannte Beschichtungs- und Kaschierungsverfahren Laminate und Mehrschichtverbunde hergestellt werden.

16. Verfahren gemäß Anspruch 1 bis 13, dadurch gekennzeichnet, daß das erfindunsgemäß hergestellte Kollagenband zur Herstellung eines Wundschnellverbands oder Wundverbands mit durchlaufendem Wundkissen in Form eines Kollagenschaums verwendet wird, wobei das Kollagenband auf einen selbstklebend ausgerüsteten Träger aufgebracht, eine Abdeckung, die vorzugsweise eine Abzieh- und/oder Applikationshilfe aufweist, zugeführt und anschließend das so hergestellte Material auf das gewünschte Maß konfektioniert wird.

17. Verfahren gemäß Anspruch 1 bis 16, dadurch gekennzeichnet, daß das erfindungsgemäß hergestellte Kollagenband zur Herstellung eines Wundschnellverbands oder Wundverbands mit zentralem Wundkissen in Form eines Kollagenschaums verwendet wird, wobei von dem Kollagenband mittels Querschneider Abschnitte jeweils definierte Größe abgeteilt und auf einen selbstklebend ausgerüsteten Träger zentral aufgebracht werden, eine Abdeckung, die vorzugsweise eine Abzieh- und/oder Applikationshilfe aufweist, zugeführt und anschließend der so hergestellte Wundschnellverband oder Wundverband konfektioniert wird.

18. Verfahren gemäß Anspruch 1 bis 17, dadurch gekennzeichnet, daß alle Arbeitsschritte unter aseptischen Bedingungen durchgeführt werden und daß die erhaltenen Produkte steril sind.

19. Verwendung der Kollagenschäume nach Ansprüchen 1 bis 18 als Tupfer zur lokalen Blutstillung, als ein- oder mehrschichtige Wundabdeckung, als Nasentamponaden, als Polstermaterial, als Füllmaterial für Knochenhohlräume oder als resorbierbarer Wirkstoffträger für Implantate für medizinische Zwecke, als Tücher oder Tupfer für kosmetische Zwecke oder als saugfähige Mehrschichtunterlagen, als Windel oder Monatsbinden für Hygienezwecke.

## Claims

1. A process for the production of collagen foams in the form of continuous tapes, characterized in that
a) dissolved or dispersed collagen is processed and cast into disk molds, whereby for stabilizing the foam, during the processing of the collagen cleavage of the intermolecular bonds of the collagen is controlled such that only a low number of intermolecular bonds is cleaved and for the most part higher-molecular, water-insoluble collagen aggregates having a helical structure are retained which collagen aggregates lead to a high natural degree of crosslinking of the collagen and thus to collagen foams of high mechanical strength, and that the collagen concentration in the solution or dispersion is 0.1 to 5%-wt., preferably 0.5 to 2%-wt.,
b) the collagen solution or dispersion filled in the disk mold is shock-frozen to temperatures of -50°C and lower in order to achieve a homogeneous sponge having very fine pores, and subsequently freeze-dried under vacuum, the drying time until a product temperature of ca. 30 °C is reached lying between 12 h and 60 h, depending on the layer thickness
c) the freeze-dried collagen foam is given an exactly defined thickness, prior to its further processing,
d) continuous tapes are produced from the freeze-dried collagen disks by a mechanical peeling process.

2. A process according to claim 1, characterized in that the collagen solution or dispersion contains active substances and/or auxiliary substances.

3. A process according to claims 1 to 2, characterized in that the collagen solution or dispersion is sterile.

4. A process according to claim 1, characterized in that the disk mold, into which the collagen solution or dispersion is filled, is of optional, preferably round shape.

5. A process according to claim 1, characterized in that the disk mold is of any height between 0.5 and 10 cm, preferably between 1 and 4 cm, and of any diameter, preferably between 50 and 70 cm.

6. A process according to claims 1 to 5, characterized in that the disk mold is provided with a central opening, preferably of round shape, having an optional area, preferably between 1 cm² and 80 cm², so that the collagen foam after freeze-drying has a central hole.

7. A process according to claims 1 to 6, characterized in that a central hole is punched or cut from the collagen foam, in case that a disk mold is used for filling which is not provided with a central slot.

8. A process according to claims 1 to 7, characterized in that a disk-shaped collagen foam or several disk-shaped collagen foams are pushed vertically side by side on a hub.

9. A process according to claims 1 to 8, characterized in that continuous tapes of any thickness, preferably of 1 to 5 mm thickness, are peeled from the disk or disks on a tape peeling machine.

10. A process according to claims 1 to 9, characterized in that continuous tapes are produced from the collagen disks by means of other known processes, preferably by laser beam cut.

11. A process according to claims 1 to 10, characterized in that the collagen tapes thus produced are wound up to rolls.

12. A process according to claims 1 to 10, characterized in that the collagen tapes such produced are folded and piled in containers.

13. A process according to claims 1 to 10, characterized in that the collagen tapes thus produced are further processed in an operation step directly following the tape production.

14. A process according to claims 1 to 13, characterized in that individual sections each having a defined size are separated from the collagen tape produced according to the invention by means of a cross cutter, said sections being made-up either individually or as a plurality, or placed in a dispenser in wound-up-condition, said dispenser preferably being provided with a break-off edge.

15. A process according to claims 1 to 13, characterized in that laminates and multi-layer composites are manufactured from the collagen tape produced according to the invention and other tape-like materials by means of known coating and laminating processes.

16. A process according to claims 1 to 13, characterized in that the collagen tape produced according to the present invention is used for the manufacture of a first-aid dressing, or wound dressing with continuous wound pad in the form of a collagen foam, whereby the collagen tape is put on a carrier which has been rendered self-adhesive, a covering is applied which is preferably provided with a stripping off and/or application aid, and subsequently the material so obtained is finished to the size desired.

17. A process according to claims 1 to 16, characterized in that the collagen tape produced according to the invention is used for the manufacture of a first-aid dressing or wound dressing with a central wound pad in the form of a collagen foam, whereby sections of defined size are separated from the collagen tape by means of a cutter and are put centrally on a carrier which has been rendered self-adhesive, a covering is applied which preferably is provided with a stripping-off and/or application aid, and subsequently the first-aid dressing or wound dressing is finished.

18. A process according to claims 1 to 17, characterized in that all operational steps are carried out under aseptic conditions and the products obtained are sterile.

19. The use of the collagen foams according to claims 1 to 18 as sponges for local hemostasis, single- or multi-layer wound dressings, nose tampons, upholstery material, filling material for bone cavities, or as resorbable active substance carrier for implants for medical purposes, as cloths or sponges for cosmetic purposes, or as absorbing multi-layer underlying sheets, as diaper or sanitary napkins for hygienical purposes.

## Revendications

1. Procédé de préparation de mousses de collagène sous forme de bandes sans fin, caractérisé en ce que
a) on prépare du collagène dissous ou dispersé et on le coule en forme de disque, où en vue de la stabilisation de la mousse au cours de la préparation du collagène, on règle ou commande la scission des liaisons intermoléculaires du collagène en une manière telle que seul un petit nombre de liaisons intermoléculaires soit scindé et que demeurent conservées principalement des agrégats de collagène de poids moléculaire élevé et insolubles dans l'eau avec une structure hélicoïdale des molécules, qui assurent un degré de réticulation naturel élevé du collagène et, de ce fait, des mousses de collagène de haute solidité ou résistance mécanique et que la concentration en collagène dans la solution ou la dispersion fluctue de 0,1 à 5% en poids, de préférence 0,5 à 2% en poids,
b) on soumet la dispersion ou la solution de collagène introduite dans le moule en forme de disque à une congélation brusque ou brutale à des températures de -50°C ou encore inférieures en vue de l'obtention d'une éponge homogène à très fins pores et on la soumet ensuite à un séchage par congélation ou lyophilisation sous vide, où la durée du séchage jusqu'à atteindre une température de produit d'environ 30°C se situe entre 12 heures et 60 heures en fonction de l'épaisseur du lyophilisat,
c) on amène la mousse de collagène séchée par congélation jusqu'à une épaisseur définie avec précision avant d'en poursuivre le traitement et
d) à partir du disque de collagène séché par congélation, on fabrique des bandes sans fin par un processus de pelage en machine.

2. Procédé suivant la revendication 1, caractérisé en ce que la solution ou la dispersion de collagène contient des substances actives et/ou adjuvantes ou auxiliaires.

3. Procédé suivant l'une quelconque des revendications 1 et 2, caractérisé en ce que la dispersion ou la solution de collagène est stérile.

4. Procédé suivant la revendication 1, caractérisé en ce que le moule en forme de disque dans lequel on introduit la dispersion ou la solution de collagène est de n'importe quelle forme, de préférence ronde.

5. Procédé suivant la revendication 1, caractérisé en ce que le moule en forme de disque est de n'importe quelle hauteur comprise entre 0,5 et 10 cm, de préférence entre 1 et 4 cm et a n'importe quel diamètre, de préférence compris entre 50 et 70 cm.

6. Procédé suivant l'une quelconque des revendications 1 à 5, caractérisé en ce que le moule en forme de disque comporte un évidement central, de préférence de conformation ronde, d'une surface quelconque, de préférence comprise entre 1 cm² et 80 cm², en sorte que la mousse de collagène après séchage par congélation présente un trou central.

7. Procédé suivant l'une quelconque des revendications 1 à 6, caractérisé en ce que, dans le cas où on utilise un moule en forme de disque sans évidement central lors du remplissage, on découpe ou estampe un trou central dans la mousse de collagène après le séchage par congélation.

8. Procédé suivant l'une quelconque des revendications 1 à 7, caractérisé en ce que l'on glisse une mousse de collagène en forme de disque ou plusieurs mousses de collagène en forme de disque l'un à côté de l'autre verticalement sur un axe d'enroulement.

9. Procédé suivant l'une quelconque des revendications 1 à 8, caractérisé en ce que l'on prélève par pelage à l'aide d'une machine de prélèvement ou de pelage de bande, des bandes continues à partir du ou des disques, de n'importe quelle épaisseur, de préférence d'une épaisseur de 1 à 5 mm.

10. Procédé suivant l'une quelconque des revendications 1 à 9, caractérisé en ce que, à l'aide d'autres techniques connues, on fabrique des bandes continues à partir des disques de collagène, de préférence par coupe au rayon laser.

11. Procédé suivant l'une quelconque des revendications 1 à 10, caractérisé en ce que les bandes de collagène ainsi obtenues sont enroulées sur des rouleaux.

12. Procédé suivant l'une quelconque des revendications 1 à 10, caractérisé en ce que les bandes de collagène ainsi obtenues sont pliées et empilées dans des récipients.

13. Procédé suivant l'une quelconque des revendications 1 à 10, caractérisé en ce que les bandes de collagène ainsi obtenus sont soumises à un traitement subséquent au cours d'une étape de travail qui suit directement la fabrication des bandes.

14. Procédé suivant l'une quelconque des revendications 1 à 13, caractérisé en ce que de la bande de collagène fabriquée conformément à la présente invention, on prélève, à l'aide d'un dispositif de coupe transversal, des sections individuelles d'une grandeur à chaque fois définie, que l'on peut introduire par section ou par plusieurs sections ou à l'état enroulé dans un distributeur qui, de préférence, présente une arête de prélèvement par déchirement.

15. Procédé suivant l'une quelconque des revendications 1 à 13, caractérisé en ce qu'à partir de la bande de collagène fabriquée conformément à la présente invention et d'autres matériaux en forme de bande, on fabrique des pansements multicouche par des processus de revêtement et de lamification connus.

16. Procédé suivant l'une quelconque des revendications 1 à 13, caractérisé en ce que l'on utilise la bande de collagène fabriquée conformément à la présente invention pour la fabrication d'un pansement rapide pour plaies ou d'un pansement pour plaies avec tampon de plaie continu sous forme d'une mousse de collagène, où la bande de collagène est appliquée sur un support rendu autocollant, on amène un recouvrement, qui, de préférence, présente une aide d'arrachement et/ou d'application et on confectionne ensuite le matériau ainsi fabriqué à la mesure souhaitée.

17. Procédé suivant l'une quelconque des revendications 1 à 16, caractérisé en ce que la bande de collagène fabriquée conformément à la présente invention est utilisée pour la fabrication d'un pansement rapide pour plaies ou d'un pansement pour plaies à tampon de plaie central, sous forme d'une mousse de collagène, où on prélève de la bande à collagène, à l'aide d'un dispositif de coupe transversal, des sections de la grandeur à chaque fois définie et on les applique centralement sur un support rendu autocollant, on amène un recouvrement qui présente une aide d'arrachement et/ou d'application et on confectionne ensuite le pansement rapide pour plaies ou le pansement pour plaies ainsi fabriqué.

18. Procédé suivant l'une quelconque des revendications 1 à 17, caractérisé en ce que toutes les étapes opératoires sont entreprises dans des conditions aseptiques et en ce que les produits obtenus sont stériles.

19. Utilisation des mousses de collagène suivant l'une quelconque des revendications 1 à 18 à titre de tampons en vue d'une hémostase locale, à titre de recouvrement de plaies ou blessures à une ou plusieurs couches, à titre de tampons pour tamponner le nez, à titre de matériau servant de tampon, à titre de matériau de charge pour des espaces creux des os, ou à titre de supports de principe actif résorbables pour des implants à des fins médicales, à titre de serviettes ou de tampons à des fins cosmétiques, ou à titre de bases multi-couche aspirantes ou absorbantes, à titre de couches ou de bandes cataméniales à des fins hygiéniques.
